# EUROPEAN PATENT APPLICATION

(11) **EP 4 248 926 A1**
(43) Date of publication of application: **27.09.2023**
(21) Application number: 22164271.3
(22) Date of filing: 25.03.2022
(51) Int. Cl.: A61F 13/15, A61F 13/505

(54) **HYGIENE ARTICLE WITH EASY TO CHANGE ABSORBENT CORE**

(71) Applicant: W. Pelz GmbH & Co. KG, 23812 Wahlstedt/Holstein (DE)
(72) Inventor: RÖTTGER, Henning, 24568 Kaltenkirchen (DE); WALLERT, Matthias, 23795 Bad Segeberg (DE)
(74) Representative: Plischke, Manfred

(57) **Abstract**

The present invention is a hygiene article system with particular re-usable chassis and a replaceable absorbent pad.

## Description

### Field of the Invention

The present invention relates to a hygiene article system with particular re-usable chassis and a replaceable absorbent pad.

### Background

Apart from washable cloth diapers, two-piece systems comprising a reusable and washable outer layer and a disposable insert have long been used. Over the last decades, disposable diapers have significantly improved both ease of application and liquid handling performance, which further led to healthier skin of a wearer even at extended wear times, not only for babies but also for incontinent adults as well as for menstruating women. Especially for the latter two categories, absorbent pads are broadly used for lower liquid handling requirements, such as light to medium incontinence or lower menstrual flow conditions, whereby such pads are typically inserted into regular underwear, optionally attached thereto by releasable adhesive, or - mostly in hospitals or care facilities - elasticated pants. In particular for mobile people, discreetness during wear times and ease of replacement become highly relevant. However, with an increased environmental awareness, disposable products have come under scrutiny for using non-renewable resources and/or for an increase carbon footprint in the overall lifecycle, including treatment of the used products.

It is also known to design products in particular for menstrual products or very light incontinence products, being washable with a slightly increased absorbent capacity in the crotch region so as to accommodate small amounts of exudates. Such products may comprise a hydrophilic wad of fibers in the crotch region, optionally in combination with a thin protective coating or film to reduce the risk of soiling of the clothing. Such products may be laundered and reused many times, and as such also allow the use of more expensive materials, such as textile extensible materials, such as knitted cotton, as well known from conventional underwear, or elastic materials, such as rubber, preferably synthetic rubber, or synthetic elastic materials, known as LYCRA ^{™} and the like.

However, the use of such products is limited for several reasons: First, de facto the product has to be washed, at least coarsely rinsed, and dried just after being used. Otherwise, if left wet, not only unpleasant smell may develop upon wet storage, but also bacteria may develop, that will require even harsher washing conditions. To avoid bacterial growth, it is quite common to use textiles treated with biocides for such washable products having the downside of potentially having a negative impact on skin health by destroying the natural bacterial climate of the skin. Furthermore, biocides also have a negative impact on the environment if they partly get washed off during washing of the product or after disposal.

Further, the absorbent capacity is limited, as the hydrophilic wadding does not exhibit high liquid absorbency, as otherwise also the drying may become inefficient. Superabsorbent materials, as commonly used in single use products, not only dry very slowly or may even lose their absorbency properties upon washing, but also are typically available as granules, as may result in a harsh feel or hand, with other application forms such as fibers or printing onto fibrous carrier not being broadly available yet.

Even further, at away-from-home activities, the same difficulties for changing a product arise as described for one-piece articles in the above, further aggravated by the need to stash the loaded product away until being home again.

Further, with regard to environmental compatibility, the re-usable and washable products require an increased use of energy (e.g., for operating equipment, heating laundry water, and treating wastewater) and chemicals (e.g., detergents and water treatment agents), such that analysis concerning which alternative is more "environmentally friendly" is complicated, and undisputed conclusions either way do not yet appear to exist.

In view of the concerns set forth above, it would be advantageous if a wearable absorbent article were available that provides advantages afforded by both disposable and reusable diapers, while reducing the respective disadvantages of these alternatives. It also would be advantageous if a construction were provided that could simplify manufacturing processes and/or reduce costs. Henceforth several approaches are known to improve usefulness, costs, and environmental compatibility with systems that combine a more efficient disposable absorbent core with a functional reusable chassis.

US6142983 discloses a disposable absorbent article with an outer cover, an absorbent chassis and an absorbent support member which is configured to maintain the absorbent chassis in close contact with the wearer's body in use. US20060247599 refers to an absorbent garment having a garment-like outer shell and an absorbent assembly positioned therein. WO1995010992A1 discloses a diaper assembly including a washable over-garment and a reusable moisture proof sheet positioned on the inner surface side of the garment.

In EP2376046 wearable absorbent article to be worn by a wearer about the lower torso is described, comprising a re-useable chassis adapted to receive an absorbent insert therein. The chassis comprises insert fastener components and the absorbent insert comprises a pair of elasticized standing cuffs.

Along these developments, the commercially available PAMPERS ^{™} Hybrid design provides baby diapers with a reusable chassis and a disposable absorbent core - just as at the beginning of diapering, but with more modern materials, especially superabsorbent materials.

However, all the described approaches and in particular the systems with separate absorbent pads still suffer from poor positioning of the article relative to the bodily exudates releasing organs, be these the labial regions for women, the penis region for men or the anal region for either gender. Whilst current single piece articles consisting of an absorbent core to handle the discharged fluid and a "chassis" firmly connected thereto generally allow to provide adequate and sustained fit during use, the disposability requires manufacturers to balance cost versus performance - both for the "chassis" materials, whereby for example use of less elastic materials may deteriorate fit, but also for the absorbency, where use of the most cost effective designs and materials often results in bulkier or poorer performing products.

Further, there remains the difficulty of removing the absorbent insert, when loaded. This is particularly relevant for mobile adults, especially for away-from home situations, such as public toilets or restrooms. Especially single piece articles can only be replaced by essentially complete undressing, which is not only inconvenient but also embarrassing the user. Further, this is considered as non-hygienic by the user, as the inner surfaces of a fresh article, which are intended to be in touch with the private parts, can get soiled by shoes or feet in touch with a soiled floor while putting on the fresh article, in particular in case of pant-style articles. In case of pads, which are to be worn in the underwear, the issue remains that they may be difficult to be removed because of the adhesive attachment, often complicated by so called wings, folded around the underwear in the crotch region. Further, it may be difficult to position the replacement pads properly, regardless if doing so when being seated, e.g. on the toilet, or in a standing position. During this procedure the inner surface of the underwear and new pad can get in touch with the fingers which often cannot get cleaned properly while away from home which is considered as non-hygienic by the user.

Further delicate undergarment may be compromised by the adhesives and/or may not allow adequate cleaning, e.g. washing at higher temperatures.

Overall, there remains the need for products that present an environmentally acceptable approach securely capture bodily exudates with a well-fitting and discreet appearance similar to regular underwear, but that allow easy and non-embarrassing change, if needed. Also handling by a caretaker (e.g. parent for babies) should be significantly simplified if only urine loading has occurred.

Thus, the present invention aims
- in a first aspect at a chassis article, as may be re-usable;
- in a second aspect at an absorbent pad, as may be disposable; and
- in third aspect at kit of chassis article and an absorbent pad.

### Summary

In a first aspect, the present invention is an article chassis for being worn on the lower torso of a wearer exhibiting a user-oriented surface and an opposite outer surface, which comprises
- at least one liquid intake zone adapted to cover a region of the exudate releasing body openings, comprising an intake zone material exhibiting, when tested on the surface adapted to be in contact with the user
   - a repeated liquid strikethrough value according to NWSP 070.7 R0 (15) of less than 50 sec, preferably less than 25 sec, preferably less than 15 sec and most preferred less than 10 sec and
   - a wetback value after repeated liquid strikethrough of less than 10g, preferably less than 5 g, preferably less than 1 g, more preferably less than 0.5 g, even more preferably less than 0.3 g, according to NWSP 070.8 R0 (15);
- a main chassis region preferably circumscribing the at least one liquid intake zone;
- optionally a transition region, connecting intake zone and main chassis region.

At least one of the main chassis and the transition region comprise material exhibiting a vertical capillary wicking height of less than 10 mm, preferably less than 5 mm, more preferably being essentially non-wicking, according to AATCC 197, 2018 Edition, 2018 - Test Method for Vertical Wicking of Textiles, optionally being water repellant or being liquid impermeable. The article chassis further comprises one or more combination element(s) for combination with an absorbent core on the outer surface in contact with the liquid intake zone, selected from the group consisting of
a) the outer surface being at least partially adapted to engage with mechanical fastener and/or connect with adhesives and/or van-der-Waals forces based micro-structured fastener film;
b) pockets positioned on the outer surface covering at least a portion of at least one intake zone, optionally made of an elastic material, optionally being liquid impermeable at the surface opposite to the intake layer;
c) straps positioned on and permanently connected to the outer surface, optionally being
   c1) elastic and permanently connected at both ends
      or
   c2) permanently connected only at one end and releasably connected on the other end, preferably with mechanical fastener and/or adhesives on the other end and/or van-der-Waals forces based micro-structured fastener film.

The article chassis may further comprise one or more elements selected from the group consisting of
d) leg cuffs, preferably at an extension from base to end of more than 1 cm, preferably more than 2 cm, more preferably more than 3 cm;
e) openable side closure elements that are in a closed configuration;
f) consisting essentially of materials and construction elements that are washable at between 40°C and 90 °C;
g) comprise materials exhibiting a Moisture Vapor Transmission Rate (MVTR) of at least 5000 g/m²/24h, preferably more than 8000 g/m²/24h when tested according to "Water Vapor Transmission Rate by the Principle of Measuring the Time to Increase Humidity Lyssy/EDANA," Part 2 NWSP 070.6.R0 (15);
h) comprise materials exhibiting an air permeability of at least 0.2 m³/(m^{2∗}s), preferably more than 1 m³/(m^{2∗}s) when tested according to ASTM D 737-18 measured at 125 Pa with a surface area of 20 cm².

The article chassis may even further exhibit one or more elements selected from the group consisting of
i) a circumferential extensibility at least in a waist region of at least 10%, preferably 50% and more preferably 100% of the unstretched circumference;
j) the one or more intake zone(s) are adapted to male user, female user, or unisex;
k) the materials of the main chassis region and an intake region exhibit a difference of at least 10% of
   - repeated liquid strikethrough time (NWSP 070.7 R0 (15));
   - wetback after repeated strikethrough time (NWSP 070.8 R0 (15));
   - Vertical capillary wicking for 0.9% NaCl solution according to AATCC 197, 2018.

In a second aspect, the present invention is a hygiene article system, comprising an article chassis and an absorbent pad, wherein the absorbent pad comprises
- a topsheet with a wearer oriented surface;
- an opposite surface with a backsheet:
- an absorbent core in between comprising absorbent pad absorbent material;
- optionally comprising a pad fluid handling aid.

The absorbent pad is further adapted
- to be positioned in registry with at least a portion of the intake zone of the article chassis,
   and
- to be combined with its wearer oriented topsheet surface to the article chassis by means of the combination element(s) of the article chassis.

The absorbent pad may further comprise one or more elements selected from the group consisting of
z) a tab or a handle as removal aid
   z1) as an extension of a peripheral region of the pad; or
   z2) attached to the outer surface of the pad;
y) a finger pouch on its outer surface as application aid;
x) a removable release cover;
w) cellulosic pulp exhibiting a pH of less than 6, preferably less than 5.5 according to the "pH measurement test of cellulose", as described herein;
v) an odor reducing agents;
u) a bacterial growth inhibitor;
t) absorbent material, optionally superabsorbent material such that the absorbent pad exhibits an absorbent capacity of more than 10 ml, preferably more than 50 ml or more preferably of more than 100 ml of an aqueous 0.9% NaCl solution;
s) absorbent material, optionally superabsorbent material and optionally an acquisition aid, the article exhibiting a rewet value of at less than 10g, preferred less than 5g and most preferred less than 1g when submitted to the "Adult Incontinence Article: Rate of acquisition and Re-Wet test NWSP 070.9 R1 (15)".

The absorbent pad may even further exhibit one or more features selected from the group consisting of
r) a urine related Acquisition value of less than 300 sec, when submitted to the Urine related Acquisition and Rewet Determination Test PA07/03;
q) a urine related Rewet value of less than 10 g, preferably less than 5 g, and more preferably of less than 1 g, when submitted to the Urine related Acquisition and Rewet Determination Test PA07/03;
p) a menses related Acquisition value of at least less than 20 sec when submitted to the Menses related Acquisition and Rewet Determination Test PA 05/02;
o) a menses related Rewet value of less than 5 g when submitted to the Menses related Acquisition and Rewet Determination Test PA 05/01;
n) a surface pH value of less than about 7.0, preferably less than about 5.5, more preferably of less than about 5, when tested according to the surface pH measurement test, as described herein.

In a particular execution, the absorbent pad of the hygiene article system exhibits a size smaller than the fluid intake zone of the hygiene article chassis, preferably covering less than 90 %, preferably less than 70%, more preferably less than 50% of the fluid intake zone, optionally having a liquid permeable backsheet material.

In another particular execution, the hygiene article chassis of the hygiene article system comprises a chassis fluid handling aid, positioned in direct contact with the liquid intake zone, and/or on a user oriented surface of a pocket.

In yet a further particular aspect, in a hygiene article system according to the present invention, the chassis fluid handling aid is adapted to release more than about 50%, preferably more than about 85%, more preferably more than about 90% of its liquid load to the absorbent pad material, when submitted to the "Liquid release of fluid handling aid material to ultimate liquid storage material of absorbent pad" test, as described herein.

### Brief Description of the Figures

Fig. 1A and B depict specific elements of absorbent systems according to the present invention.
Fig. 2A to D depict specific elements of an absorbent pad according to the present invention.
Fig. 3A to E depict specific combinations of a chassis and an absorbent pad according to the present invention
Fig. 4A to C depict specific executions of a combination of a chassis and an absorbent pad according to the present invention.

The figures are schematically only, and not to scale. Same numerals refer to same or equivalent features or elements, single (') or multiple (", '", ...) apostrophes indicate duplicate features, such a left and right or front and back, etc..

### Detailed description

Within the present context, absorbent hygiene articles are intended to be worn on the lower torso of a human wearer of any age, adapted to receive bodily exudates, especially liquid exudates, such as urine, menses, or faeces, as are released in the vaginal region, or from the penis, or from the anus, towards the "intake zone" of the article positioned adjacent to the exudate releasing organ. Absorbent hygiene articles comprise an "absorbent core" for handling the bodily fluids, i.e. acquiring, distributing and storing these therein, and a "chassis" for holding the absorbent core in place on the body of a wearer. Absorbent articles may be donned and doffed by the user him- or herself, or by a care-taker, such as a parent for babies.

Hygiene articles can be classified as
- "pants style" articles, which enclose the waist of the wearer firmly connected like a belt, but which may include means for tearing a side seam open for easy doffing. Typically, such articles are stretchable or at least extensible for good fit and for easing donning,
- or "taped diapers" that comprise fastening means for connecting the front and rear parts around the waist at donning, though these may also be pre-closed during manufacturing, such that they have a pants like appearance, but are easy to be opened and re-closed, if necessary.

In order to adapt to the complex body contours, the chassis often exhibit "elasticity", such as induced by elastic elements like rubber, preferably synthetic rubber, strands, including LYCRA^{™} or similar, but also elastomeric polyolefins, as in films or non-wovens. In addition, or alternatively, the chassis may comprise structurally "extensible" materials, such as well-known e.g. from knitted cotton underwear, wherein the knitted yarns are essentially non-elastic, but the knitted structure can readily conform to the body contours, albeit at retractive forces that are typically lower than for elastic materials.

An "absorbent pad" essentially consists of the absorbent core between a topsheet and a backsheet, and may be hold in place on the body by other means, such as regular underwear, optionally aided by non-permanent adhesives and/or "wings" as may be folded around the crotch portion of the underwear.

The term "permanent" refers to a feature that remains essentially unchanged during the intended use, and as such may be a connection, that can only be disconnected destructively, or a treatment, such as a hydrophobization, that remains during the intended uses, although its effect may be reduced.

A "single piece diapers" comprise an absorbent core firmly connected to a "chassis" by which the absorbent core is held in place on the body of the wearer.

For "two-piece systems", the absorbent core in an absorbent pad and the chassis are separate or at least can be readily separated, though "pre-combined systems", where the manufacturer combines the core and chassis removably, may be used. The absorbent core may be in a "replaceable" absorbent pad as can be readily removed and for which a like component or a component providing similar functionality can be substituted. The chassis may be similar to the underwear, as well known e.g. from hospital "net-pants" holding any absorbent pad more or less accurately in place. The present invention is directed to such a two-piece system, whereby, however, the pad and the chassis are particularly improved as such and even more so when cooperating.

The absorbent hygiene articles comprise an "inner" or "wearer facing layer", forming the innermost surface which may be in direct contact with the skin during use, typically formed by a skin contact sheet or "topsheet", but may also include other elements such as (single or multiple) leakage barrier cuffs or inwardly positioned chassis layers in the waist or hip regions. The absorbent hygiene article also comprises an "opposite" or "garment facing layer", with elements of the chassis that form the outer surface of the absorbent article, such as the backsheet, the side panels, the waist fasteners, and the like, when present. The term "major surface" describes surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.

Absorbent hygiene articles or elements thereof may be "disposable", i.e. intended for a single use for being discarded, optionally recycled, in an environmentally compatible and hygienic manner. Absorbent hygiene articles or elements thereof may be fully "re-usable", which are adapted to be cleaned many times, typically withstanding at least 60 times, without major deterioration of its properties and functionality, and thus withstand washing with conventional detergents, preferably at at least 60°C or more, though lower temperatures may be acceptable, if particular cleaning agents are employed, or if the materials of the articles have been particularly treated, such as by permanently incorporated biocides. "Limited re-usability" refers to articles that can be cleaned multiple times, though have a higher wear than the fully re-usable ones, and/or may be discarded like disposable articles.

Examples for fully re-usable articles are so-called "menstruation pants" that exhibit a low to moderate absorbency as may be achieved by hydrophilic fibers, such as cotton fibers or yarns, at a higher basis weight in, optionally also around, the intake zone.

The above mentioned "net pants" for hospital use typically exhibit a limited re-usability, as they can withstand washing conditions, at least for a few times, but may also be discarded, e.g. if heavily soiled by faeces, and may also be torn open for ease of removal in such cases.

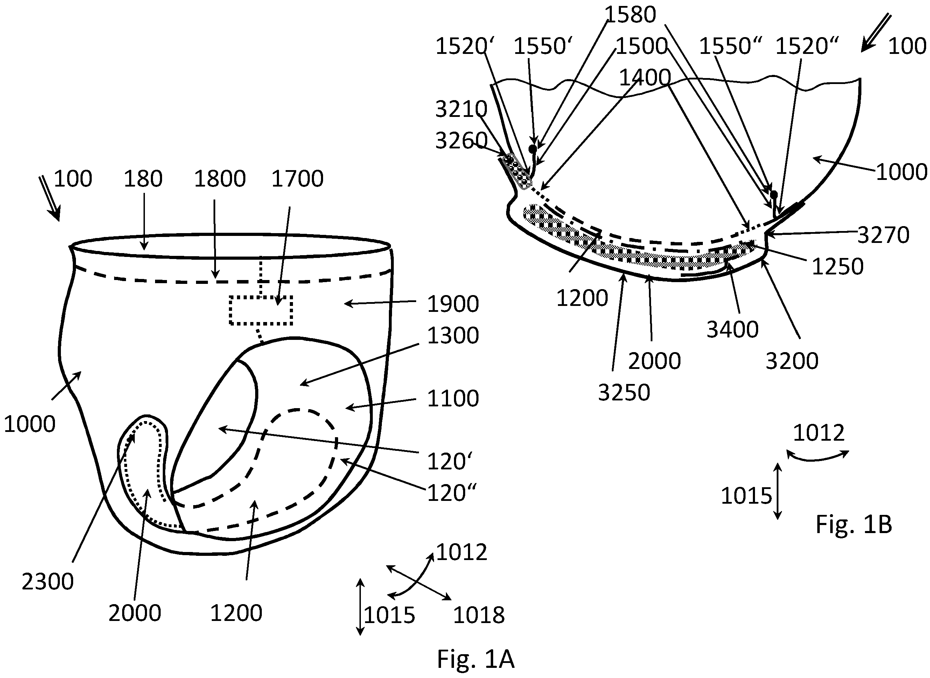

The elements of the present invention as well as their functioning is now explained by referring to Fig. 1 to 3, whereby the shown executions should not be seen limiting. Also, certain features or elements may be used independently from the other elements shown in the figures.

When describing specific properties of materials and/or of elements, reference is made to the "test Method" section herein below, wherein the determination of the respective parameter by specific test methods is described in detail.

In a first aspect the present invention is an absorbent hygiene two-piece system, or kit, 100, as exemplarily depicted in Fig. 1 in a shape as may be on a wearer, with a waist opening 180 and leg openings 120' and 120", comprising an article chassis 1000 and a separate removable absorbent pad 2000, whereby both are adapted to cooperate with each other, with details thereof being described herein after. Such a system not only offers significantly improved environmental footprint versus a one-piece system, but also allows better handling by a user.

The chassis 1000 exhibits a width (y-) 1018 and a length (x-) 1012 direction, the first corresponding to a left-right orientation on a user, the second corresponding to a line extending form the front, i.e. navel, through the crotch to the back, i.e. small of the back, of a user. A thickness (z-) direction 1015 extends perpendicularly to both, whereby the terms "upper" / "inner" and "lower" / "outer" correspond to "towards the user" and "away from the user", respectively. The skilled person will readily apply such terms also to a chassis and an absorbent pad when detached from a user, such as when manufactured or packaged.

### Chassis

The chassis 1000 may be executed in a "pants style", or - as indicated with dotted lines in Fig. 1A in an open diaper style, whereby the front and rear side portions are connected by closure elements 1700, as fixed by a user or optionally already at manufacturing ("pre-closed").

The chassis 1000 may be a re-usable and washable pants shaped article, with an inner layer 1100, which is essentially continuous but comprises a fluid intake zone 1200 and a main chassis region 1300, exhibiting a significant difference of at least one property, e.g. hydrophilicity, porosity, rewet. As depicted in the figure, the fluid intake zone 1200 may be completely circumscribed by the main chassis region 1300, though it may extend towards the perimeter of the chassis, e.g. laterally in the crotch region. Optionally there may be a transition region 1400, see Fig. 1B, where the materials of the intake zone 1200 and the main chassis region 1300, if different, may be connected, or where the change of property/ies occurs. Generally, the chassis exhibits superior fit properties, good softness, a nice hand, such as known from knitted cotton underwear.

The intake zone 1200 is made of material that allows released exudates to penetrate through and away from the wearer, but exhibits low rewet properties for a dry feel even after loading. Optionally, the intake zone 1200 may comprise further handling aids 1250, such as liquid acquisition or distribution materials, which may improve liquid uptake, but should not compromise rewet properties.

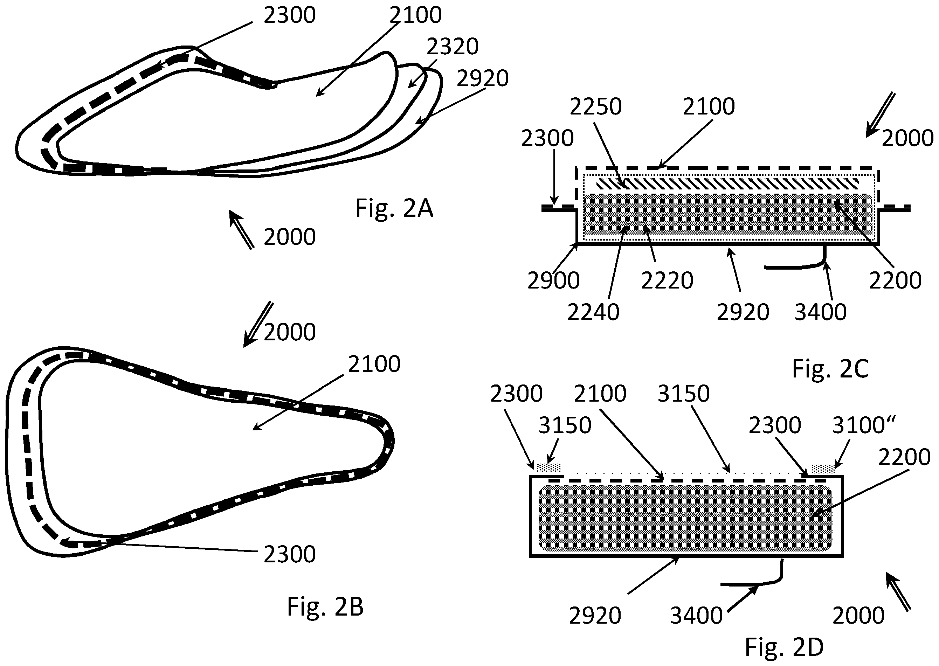

The chassis 1000 is adapted to receive an absorbent pad 2000 on its outer surface 1900, i.e. away from a wearer, whereby the application of the absorbent pad 2000 to the chassis 1000 may be made before donning of the combined chassis / absorbent pad system, but can also be readily performed after donning of the chassis without pad and/or when replacing a loaded pad. Referring to Fig. 2A to D, the absorbent pad 2000 can generally be designed as known in the art, i.e. comprising an absorbent core 2200 with absorbent material, such as pulp 2220 and preferably also superabsorbent material 2240 as ultimate liquid storage material, whilst being thin and soft or pliable for comfortable use, both when dry and loaded. It may comprise further liquid handling aids 2250, as liquid acquisition or distribution aids, as should cooperate with the ones 1250 of the intake zone of the chassis, if present. Typically, though not necessarily for all design options, the absorbent pad comprises a liquid barrier 2900 on it garment oriented side, such as backsheet 2920, and a fluid permeable topsheet layer 2100 on the user-oriented surface. The liquid barrier and the topsheet may be connected to each other along the periphery 2300 of the absorbent pad.

It is important that the connection of the chassis 1000 and the absorbent pad 2000 is executed such that good liquid transfer from the intake zone of the chassis to the absorbent pad is enabled and maintained during the full use period and therefore the chassis 1000 and the pad 2000 are adapted to each other so as to exhibiting
- non or minimally hindered liquid transfer from the intake zone 1200 of the chassis towards the ultimate liquid storage in the absorbent core 2200;
- minimal rewet, in particular in the intake zone 1200;
- good liquid sealing towards the outside of the system 100;
- easy application, sustained fixation but also easy removal especially of the absorbent pad 2000 without needing to doff the chassis 1000.

To this end, the chassis 1000 and the absorbent pad 2000 are connected to each other by connection elements 3000, which can be part of the chassis 1000, or of the absorbent pad 2000, or of both.

In a first execution, the pad may be attached to the chassis by attachment elements such as selected alone or in combination from the list consisting of:
- mechanical fasteners, whereby preferably the "looped portion" is permanently on the chassis side and soft and pliable hooks on the pad surface oriented towards the chassis;
- or van-der-Waals forces based micro-structured fastener film, such as mushroom shaped miniature piles providing non-glue related adhesion, as available under the designation "gecko tapes" ^{™} from Gottlieb Binder GmbH, Germany;
- or adhesives, preferably applied to the pad, optionally covered by a release cover, that may optionally also cover the fluid receiving surface of the pad. Preferably such a release cover can be removed after the pad is properly positioned relative to the donned chassis, e.g. by a cover removal strip.

In Fig. 2D, such connection elements 3100 are indicated along the pad periphery 2300, and more specifically as adhesive stripes 3150 on the user-oriented surface 2100.

In the execution as depicted in Fig. 2D, the absorbent pad 2000 may be designed larger than the size of the intake zone 1200, or of the transition zone 1400, preferably adapted in shape, such that the pad periphery circumscribes the intake zone 1200, once applied. The backsheet 2920 of the absorbent pad, which is generally forming the outer pad surface 2900, and which may be formed of any liquid impermeable material, may extend around the sides of the absorbent pad 2000 towards the upper surface 2100 of the pad, e.g. by folding the backsheet 2920 so as to form a side leakage protection as well as the basis for the attachment elements. The connection elements 3100 may then be positioned on the overfolded backsheet 2920.

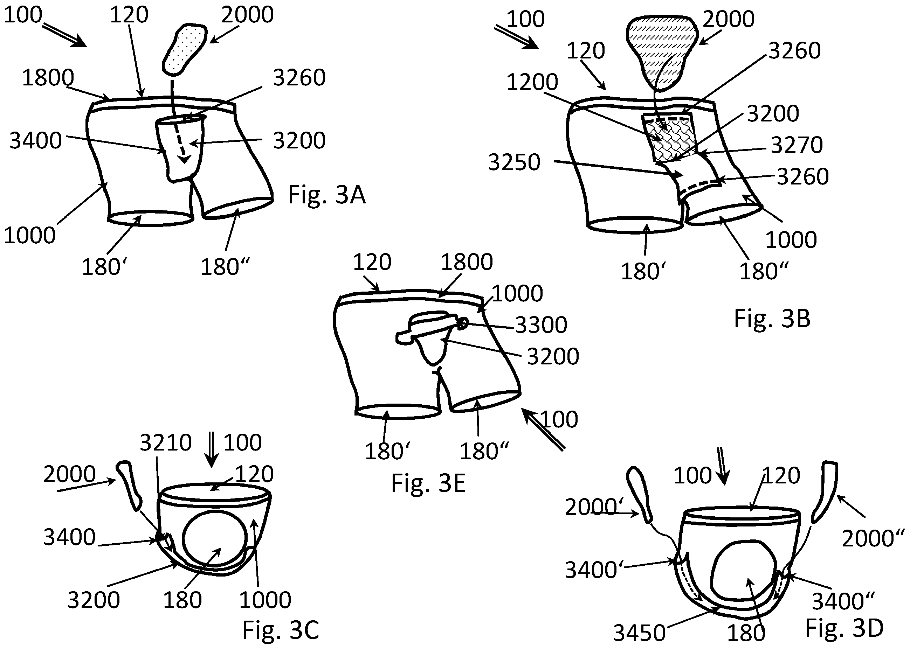

As exemplarily shown in Fig. 1B, other executions of the connection elements may be pockets 3200 of the chassis 2000 with pocket opening 3210 covering a portion of the intake zone 1200, or at least allowing access thereto, adapted to allow positioning of the absorbent pad 2000 therein. The pocket may be executed with three sides being permanently connected to the outer surface 1900 of the chassis, see Fig. 3A and 3C, or essentially only on one side in a "drop fly" style 3250, see Fig. 3B, whereby additionally attachment elements 3260 may allow closing of the drop fly for application, removal or replacement of the absorbent pad. The orientation for opening the drop-fly can vary for different design options, i.e. the hinge 3270 may be positioned along the width direction of the article, such that the drop fly can be opened "downwardly", or the hinge may be position along the longitudinal direction, such that the drop fly can be opened sideways. The pocket, in particular in the "drop-fly" execution, may comprise attachment elements 3260 for removably connecting the pocket or the drop-fly to the main chassis region.

The pocket materials 3220 may be liquid impermeable material, such as a plastic film, or film/nonwoven composite, or liquid repellant textiles or liquid permeable materials, depending on the liquid barrier properties of the backsheet of the absorbent pad.

Even further executions of the connection elements may be elastic or non-elastic straps 3300, see Fig. 3E, that are permanently or releasably connected to the chassis such that the absorbent core can be slipped underneath or be connected on its outer surface thereto.

The material of the pockets or the straps may be elastic, such that upon application it is stretched to then apply forces to the pad / chassis interface in the intake zone, thereby enhancing contact and improving liquid transfer, or even further enhance the body contact.

In an even further execution, the user oriented surface of the absorbent pad may comprise an adhesive 3150 applied to the surface, see Fig. 2D, which should be applied such that it does not overly block liquid transfer, and which preferably has a higher adhesion to the surface of the absorbent pad than to the chassis, such that it is removed together with the pad.

It should be noted that the fluid intake zone may have shapes and sizes that are adapted to the intended use, e.g. being designed for a female application, a male application or a non-gendered application. Accordingly, also the connection elements may be adapted. A drop-fly as shown in Fig. 3B may be particularly useful for a male application, as well as a pocket design with a slit-type opening positioned towards the front waist of a wearer, see Fig 3A. For a female design, a pocket with a pocket opening positioned more towards the crotch region may be appropriate, see Fig. 3C, or even a "tunnel pocket", see Fig. 3D, with pocket openings in the front and in the back of the chassis article, as may be particularly useful, when also the anal region should be covered, optionally by using a secondary absorbent pad.

Optionally, the chassis may comprise further leakage barriers 1500, see Fig. 1B shown as a front 1500' and a rear 1500" barrier, positioned longitudinally outside of the intake zone 1200, such as well-known from conventional articles as "barrier leg cuffs". Such leakage barriers are connected at their base 1520', 1520" to the inner surface of the chassis, extend at least 1 cm, or 2 cm, or 3 cm away from the base, and may end with an elastic thread 1550', 1550" at the end 1580 opposite of the base. Such leakage barriers may also exhibit a primarily longitudinal extension and extend over the full length of the chassis. Such leakage barriers may be made of any liquid impermeable material, preferably hydrophobic nonwoven or textile, more preferably liquid impermeable up to a hydro-head of more than about 10 mm, or more than about 20 mm, or more than about 40 mm when measured according to EDANA NWSP 080.6 R0 (15) "Evaluation of Water Resistance (Hydrostatic Pressure)".

Having described the hygiene article system with its key elements chassis and absorbent pad in general terms and with a focus on the interaction of the elements, the following will describe particular or exemplary executions and features of these elements, still referring to the Fig. 1 to 4. The skilled person will readily realize that various specific features of the hygiene article chassis and the absorbent pad may be combined for a particular article chassis, or a particular pad, or a particular system comprising a particular article chassis and a particular absorbent pad. The size and shape of chassis should be adapted to the intended use and cover the respective exudate releasing regions of the wearer, as described in the above. At least for these regions a tighter fit is preferred, like briefs, whilst outside thereof in the main chassis regions a wider fit, such as of "boxer shorts" is acceptable.

The chassis should be designed to be readily and easily donned and doffed. It may be executed as a "taped system" that comprises fastening means for connecting the front and rear parts around the waist, which are preferably pre-closed during manufacturing, such that they have a pants like appearance, but are easy to be opened and re-closed, if necessary. In an often preferred execution, the chassis is designed in a "pants style", which encloses the waist of the wearer firmly connected like a belt, but which may include means for tearing a side seam open for easy doffing. Typically, such articles are stretchable or at least extensible for good fit and for easing donning.

In order to allow easy donning and also to maintain good adaptation to body contours, suitable materials may exhibit an extensibility or elasticity, of at least about 5 %, preferably of more than about 10 % or more than about 30% or more than about 50 %, relative to the un-extended length. Preferably, the materials exhibit a low hysteresis, so the material gets back in shape after stretching, e.g. when tested according to the "tensile testing NSWP 110.4, and a low degree of "set", so as to maintain the shape during use. Preferably, retractive force are balanced so as to maintain the article in its position, even upon loading, without impacting the wearer unduly, such as red-marking would do.

The chassis may also comprise different regions of elasticity and extensibility, for example the main chassis region may exhibit extensibility as may be provided by machine knitted cotton or blends of cotton and elastic synthetic fibers. Typical blend ratios are in the range of 70% to 98% cotton and 2% to 30% elastic fibers. Instead of cotton also blends of cotton and synthetic fibers like PET and PA may be used. The chassis article may further comprise an elastic waist band 1800, see Fig. 1A, that may exhibit different elastic properties than the main chassis material. Preferably, the chassis exhibits a circumferential extensibility at least in the waist region of at least 10%, or more than 50%, or more than 100%, relative to the unstretched circumference.

It is highly preferred that the chassis, at least the main chassis region, is "breathable" (air permeable) by exhibiting air permeability values of more than about 0.2 m³/(m^{2∗}sec), or more than about 1.0 m³/(m^{2∗}sec), when tested according to ASTM D737.18, such as can be achieved by
- textile material, such as knitted or woven materials, e.g. double or fine rib or Jersey, as known from conventional cotton or Rayon/Viscose fibers or blends which may contain elastic fibers like Lycra, such as without limitation
   - 100 % knitted cotton, such as used in White sports briefs with double-rip mesh - Original Classics; Product number: 005044-100, of Schiesser GmbH, Germany;
   - knitted mix of 95% natural organic cotton and 5% spandex, such as used in Boxer briefs organic cotton dark blue - 95/5 Product number 174004-803, of Schiesser GmbH, Germany;
   - knitted mix of Polyamide 70% Elastane 30%, such as used in Maxi-Slip Microware white Invisible Soft, Product number: 166916-100 of Schiesser GmbH, Germany;
   - knitted Modal 35%, Cotton 35%, Polyamide 20%, Elastane 10%, such as used in Boxer briefs modal Hollow Air technology fine heather aquamarine - Pique; Product number: 176695-813 of Schiesser GmbH, Germany;
- non-woven materials, such as spunbonded non-woven materials, e.g. polypropylene webs with fibers of about 1.7 dTex to about 3.0 dTex; or carded nonwoven materials using blends of fibers which may be bonded by hydroentanglement;

Also vapor permeable film materials may be employed, which may exhibit a vapor permeability of more than 5000 (g/m²/24h), or more than about 8000 or more than about 10000 (g/m²/24h) according to "Water Vapor Transmission Rate by the Principle of Measuring the Time to Increase Humidity Lyssy/EDANA," Part 2 NWSP 070.6.R0 (15).

Preferably, suitable chassis should be washable, i.e. withstand at least one, or at least 10, or at least 60 wash cycles in a conventional household washing machine, e.g., Miele WCA030 WCS-type of Miele *&* Cie.KG, Germany, at at least 40°C, preferably 60°C, and 1400 rpm spinning speed. Preferably, suitable chassis should be dryer or tumbler compatible, at least under "gentle drying" conditions. Optionally, the chassis materials may be treated to reduce bacteria or odor generating effects.

The intake zone should differ from the main chassis region in at least one property, preferably more than one, that is relevant for liquid handling, which may be selected from the group consisting of
- hydrophobicity / hydrophilicity;
- porosity or aperturing;
- surface topography, such as embossing, such as of bending lines, or channel structures;
- material composition, thickness, densities, basis weights;
- rewet and/or fluid acquisition performance.

As particular examples, the intake zone may be made by modification of the main chassis material, such as by applying a particular treatment, such as a hydrophobization and/or perforation or a hydrophilization in case of a hydrophobic chassis material, or may be a different material connected in a transition zone 1400 to the main chassis material by conventional means, such as without limitation stitching, gluing, and thermobonding.

The intake zone or the materials comprised in the intake zone may exhibit the following properties:
- The material should be hydrophilic according to the Single Load Drop Test with a penetration time of less than 30 sec, preferred 15 sec and most preferred less than 5 sec or even spontaneously penetrating the material.
- The "Nonwoven Run Off' values according to EDANA NWSP 080.9.R0 (15) are preferably less than about 50 %, or less than about 25 % or less than about 10 %, or even essentially 0 %.
- "Air Permeability of Nonwoven Materials" according to ASTM D 737-18 (measured at 125 Pa with a surface of 20 cm²) preferably more than about 0.5 m³/(m^{2∗}s), or more than about 1.0 m³/(m^{2∗}s) though typically less than about 3.0 m³/(m^{2∗}s).
- Aperturing, such as by apertures exhibiting a size corresponding to a circle having a diameter of more than about 0.25 mm, or of more than about 0.5 mm, or of more than about 1 mm or even more than about 5 mm, and typically less than about 15 mm or less than about 10 mm, whereby the apertures may also exhibit a three-dimensional funnel-like shape;
- "Repeated Liquid Strike-Through Time (Simulated Urine)", EDANA NWSP 070.7 (3 times 5 ml 0,9% NaCl) of less than 50 s, or less than 25 s. or less than 15 s or less than 10 sec
- "Wetback After Repeated Strike-Through Time (Simulated Urine)" EDANA NWSP 070.8 of less than about 10 g, or less than about 5 g, or less than about 1 g or even less than about 0,5 g. Suitable materials for the intake zone may be made of a wide choice of material compositions, like nonwoven materials, such as spunbonded, carded, air-laid webs comprising alone or blended with each other
   - hydrophilic synthetic fibers, such as comprising polyolefins, polyester, polyamides, optionally including a surface treatment, optionally a permanent surfactant;
   - natural fibers, such as cellulosic fibers like cotton, hemp, flax, bamboo, or silk;
   - modified natural fibers such a cellulose based, cotton, hemp, flax, bamboo, with hydrophobic treatment;
   - regenerated natural fibers, such as cellulose, e.g. viscose, rayon, lyocell based materials, optionally treated, such as hydrophobically treated as know from OLEA ^{™} fibers of Kelheim Fibers, Germany.

Other suitable materials include textile webs, such as knitted, woven, or net structures, of threads or filaments as may be made from materials as also may be used for non-wovens.

Also apertured films and film/textile laminates, in particular apertures films exhibiting three dimensionally shaped, funnel-like apertures, optionally combined with a fiber layer on the user-oriented side, may be used.

Exemplary materials suitable for the intake zone are
- hydrophilically treated polypropylene spunbond webs, e.g. PS6KW-110018NN 18 g/m² PP Spunbond offered by Fitesa Germany GmbH;
- hydrophilically treated carded thermobonded polypropylene nonwoven e.g. pph 18 g/m² offered by pely-tex GmbH & Co. KG, Germany;
- hydrophilically treated carded through-Air Bonded nonwoven like pelyloft 25 g/m² offered by pely-tex GmbH & Co. KG, Germany;
- Spunlaced cotton nonwoven such as PurCotton 35 g/m² offered by Winner Medical, Co., Ltd, Shenzhen, China;
- Spunlaced cotton nonwoven with hydrophobic treatment and apertures e.g. with a diameter of 5 mm, e.g. offered as PurCotton by Winner Medical, Co., Ltd, Shenzhen, China;
- Polyester fabric, such as COOLMAX^{®} or COLLMAX^{®}FRESHFX^{™} offered by Orneule OY, Finland;
- Perforated Polyamide fabric with apertures, e.g. with a diameter of about 5mm, e.g. offered by Orneule OY, Finland;
- Polypropylene fabric (apertured) e.g. POLYCOLON^{®} offered by Orneule OY, Finland;
- Polyester knit, e.g. SPORTS KNIT, 2992 170 g/m² offered by Orneule OY, Finland;

In addition to function as a skin contacting layer, the material of the intake zone the handling may comprise by chassis fluid handling aids, see Fig. 1B, 1250, such as particular webs - or z-directionally 1015 arranged sub-layers of a web - that exhibit different properties, e.g. the uppermost user oriented layer may be exhibit superior softness, skin friendliness or other properties at cost of e.g. fluid uptake speed for higher loads. Then a second or sub-layer may function as a liquid acquisition aid - adapted to the absorbency properties of the absorbent pad. Between the intake zone and the main chassis region there may be a transition region 1400, see Fig. 1B, wherein the properties may gradually or stepwise change. The transition zone may also exhibit properties different from the intake zone and the main chassis region, e.g. being executed as a fully hydrophobic barrier between the intake zone with a balanced hydrophobicity and the main chassis region as may be hydrophilic.

An example for a material used for the transition region is a Polypropylene fabric (hydrophobic) e.g. POLYCOLON^{®} available from Orneule OY, Finland.

The absorbent pad comprises a topsheet 2100, a backsheet 2920, and an absorbent core 2200 (see Fig. 2) z-directionally interposed there between.

The topsheet is adapted to contact the chassis at least in the intake zone and preferably also in the transition zone, if present. The topsheet may be a conventional topsheet as used for absorbent articles, e.g. 10 g/m² spunbonded PP NW, 1.5 to 2.0 dTex.

The absorbent core may comprise an acquisition / distribution layer (ADL) 2250, instead of or additionally to a fluid handling aid of the chassis 1250, if present. Such an acquisition material is well known to a person skilled in the art, but should be adapted to the particular intended use. An exemplary execution for an acquisition material a through-air bonded Acquisition layer, e.g. Pelyloft 30, as available from Pely-Tex GmbH &Co KG, Germany or DRY WEB TDL2 (60 g/m²) available from TWE Group, Germany

The absorbent core further comprises absorbent material, preferably including superabsorbent polymer (SAP) material, as may be combined with fibrous material, e.g. cellulosic pulp.

The absorbent core may be formed in-situ by combining SAP powder with fibrous material, such as pulp or synthetic webs, preferably stabilized by adhesives, or thermofusion, such as ultrasonic bonding, as well known in the art.

In particular when intended and adapted to female users, it may be preferred that the absorbent core allows to maintain a natural pH in the vaginal region of around 5.5, by exhibiting a pH value of less than 6 or less than 5 or even less than 4 after loading the absorbent core with six fold of its dry weight with 0.9% NaCl solution and measuring the pH by pressing a pH indicator paper (MERCK 1.09527.0001) against the surface of the wetted absorbent core at the point where the NaCl solution has been applied to, e.g. by comprising particular pulp such as "Bliss ^{™} pulp of International Paper Co. USA. Maintaining a pH below 6 in the absorbent core is beneficial to mask odor based on ammonia or TMA (Tri-Methyl-Amin) typical for menstrual fluid and urine. Furthermore, the low pH inhibits the growth of bacteria similar to the protective acid mantle of the human skin. This allows to relinquish the use of biocides as commonly used in washable menstrual pants.

The absorbent core may be pre-formed such as available under the trade designation eCore from Glatfelter Falkenhagen GmbH, Germany, or MM150.SMT.CSN00.V03.F from Glatfelter Falkenhagen GmbH, Germany, or Gelok 15040-71 S/S of Gelok Int. Corp., Dunbridge, USA, or C1250956 NovaZorb^{®} from Engineeered Absorbent Materials (EAM), USA, or SuperCore SCP-200-TCF from McAirlaid's Vliesstoffe GmbH, Germany.

Alternatively, the absorbent core can be made in-line of fluff fibers or a blend for SAP (Super Absorbent Polymer) and fluff-pulp fibers formed in-line using hammer mills and drum formers on a converting line, or by combining SAP particles with synthetic fibers.

The absorbent core is preferably thin, preferably exhibiting a dry thickness of less than about 5 mm, or less than about 3 mm, or less than about 2 mm, but typically more than about 0.1 mm. The absorbent core may exhibit an overall liquid retention capacity which does not need to be maximized, as the absorbent pad can be easily replaced without changing the chassis.

Thus, the overall liquid retention capacity should be more than about 10 ml, or more than about 20 ml, or more than about 50 ml, or more than about 100 ml, but typically less than about 500 ml, or less than about 300 ml, according to "Centrifuge Capacity" (modified MWSP 241.0) determination, as described below.

In addition, a high local basis capacity, expressed as absorbent capacity per unit area, should not be excessive as otherwise it may result in an undesired bulky wearing impression.

This may be achieved, if the absorbent core exhibits a basis capacity of more than about 2000 ml/m², or more than about 4000 ml/m², or more than about 7000 ml/m². but less than about 16000 ml/m², or less than about 13000 ml/m², or less than about 10000 ml/m², calculated from the above reference centrifuge capacity according to modified EDANA NWSP 241.0.

Optionally, the absorbent core may comprise odor masking or reducing agents, as may be perfumes, odor adsorbents, or bacterial growth inhibitors, such as - without limitation -
- based on zinc ricinoleate, e.g. Zinc ricinoleate Methylglycinediacetic acid, N,N-BIS(carboxylatomethyl)alanine trisodium salt (MGDA) as commercially available under the designation FRANCHEM^{®} Sorb 30 B from Surfachem Deutschland GmbH;
- comprising Cyclodextrin e.g. CAVAMAX^{®} from Wacker Chemie AG, Germany;
- comprising silver ions, as available in silver chloride, optionally as nanoparticles. Optionally, an employed superabsorbent polymer may be modified for an enhanced odor control function, e.g. SAP type OC-301 supplied by LG-Chem, South Korea.

Optionally, a particularly modified cellulose fluff pulp may be utilized, such as exhibiting a pH of less than 6 or less than 5 or even less than 4, e.g. the above referenced "Bliss ^{™} pulp of International Paper Co. USA or Supersoft^{®} Odor Control Fluff Pulp from International Paper, USA.

The application of the absorbent pad varies from daily use, menstruation, light incontinence and heavy incontinence. Therefore, different test methods have to be applied for the determination of the acquisition and rewet depending on the target application, in particular relating to urine or menses.

Preferably, the absorbent core comprises absorbent material, optionally superabsorbent material such that it exhibits an absorbent capacity, as determined according to the centrifuge capacity test as described herein below, of more than 10 ml, preferably 50 ml or even 100 ml depending on the application (menstrual pant or incontinence pant). Preferably, the absorbent core exhibits a rewet value of at less than 10 g, or less than 5 g, or less than about 1 g when submitted to the urine related acquisition and rewet test for an absorbent pad, as described herein below.

Preferably, the absorbent core exhibits an acquisition value of at least less than 300 sec, when submitted to the urine related acquisition and rewet test for an absorbent pad, as described herein below.

In particular when intended to be used in the context of menstrual products, the absorbent core may exhibit an acquisition value of at least less than 20 sec, when submitted to the menses related acquisition and rewet test for an absorbent pad, as described herein below, or rewet value of less than about 5 g, when submitted to the menses related acquisition and rewet test for an absorbent pad.

The absorbent pad may further comprise an outer barrier 2900 preventing liquid penetration to the outside, e.g. the garments. Such a barrier is well known in the art, and may be a conventional backsheet 2920 for hygiene materials, such as film material, e.g. PE or PP films of typically less than about 25 g/m² or less than about 20 g/m², optionally in combination with a fibrous layer (also referred to as "textile backsheet"), hydrophobic NW material, e.g. PP-SMS @ 18 g/m² or SSMMSS and the like. Alternatively, the outer layer 2900 may also be liquid permeable, as will be further described herein below for designs with a chassis fluid handling aid positioned on the outer portion of a pocket.

The topsheet and the backsheet of the absorbent pad envelope the absorbent core, which can be achieved by conventional designs. In a first execution, see Fig. 2D, the backsheet may be folded around the sides of the core overlapping the topsheet along the periphery of the absorbent pad. In a second execution, the backsheet may be folded around the sides of the core, but on the chassis-oriented side of the absorbent pad it is positioned underneath the topsheet, i.e. between the topsheet and the absorbent core, whereby the topsheet may also be folded around the side towards the backsheet side to provide a more pleasant look, although in this design the topsheet should not exhibit lateral wicking.

In a further execution, see Fig. 2C, the topsheet and the backsheet extend outwardly beyond the absorbent core and may be connected in the thus formed periphery in a "butt" mode, whereby the general inner surfaces of the webs are in a touching position.

The absorbent pad may be constructed by applying conventional technologies, such as gluing, and/or thermo-bonded, optionally ultrasonic bonding.

Preferably, the absorbent pad exhibits a balanced stiffness or bending resistance, as may ease the application, in particular when inserting the absorbent pad into a pocket. In particular, for applications where the absorbent pad is to be positioned in the crotch region of a wearer, the absorbent pad should be perceived as soft, or supple, conforming readily to width-wise compression by the legs.

To this end, it may be preferred that the bending stiffness according to "Stiffness of Nonwoven Fabrics Using the Cantilever Test (INDA) NWSP 090.1.R0 (15) is
- for the longitudinal direction less than about 200 mN^{∗}cm, or less than about 150 mN^{∗}cm, or less than about 80 mN^{∗}cm, but typically more than about10 mN^{∗}cm, or more than about 20 mN^{∗}cm;
- is for the width direction less than about 150 mN^{∗}cm, or less than about 100 mN^{∗}cm, or less than about 60 mN^{∗}cm, but typically more than about10 mN^{∗}cm, or more than about 20 mN^{∗}cm,
- along the longitudinal direction higher by more than about 5 %, or more than about 10 %, or more than about 20 % compared to and based on the one in the width direction.

This may be achieved by applying particular glue or embossing lines, which may generally extend longitudinally but with a curved shape.

Optionally, the absorbent pad may comprise application aids, such as a pouch 3400 on its outer surface, adapted and sized that one or more fingers can be inserted for pushing pad into its proper position.

Optionally, for executions where absorbent pad comprises attachment elements on its surface that may have the tendency to adhere to surfaces prior to be positioned properly, such as adhesive stripes or mechanical fastener hooks, the topsheet surface of the absorbent pad may comprise a release paper, optionally comprising a removal strip allowing to pull the release paper away, once the pad is put in place.

Optionally the absorbent pad may comprise removal aid, especially for designs where the absorbent pad is positioned in a pocket of the chassis. In a first execution, when the topsheet and the backsheet are connected in a "butt" mode, whereby the general inner surfaces of the webs are in a touching arrangement, in the outer periphery of the pad, the removal aid may be an enlarged tab like portion of the periphery. Alternatively, a tab may be connected to the pad, optionally as a loop or arched handle, adapted to Finger pouch 3400 for easing application.

It is further a particular aspect of the present invention that the chassis and the absorbent pad as described in the above can be combined to a fully functional absorbent article, whereby each of the two may be replaced independently from the other, whereby the absorbent core is positioned such that the fluid receiving surface is oriented to and in good liquid contact with the intake zone of the chassis. "Good liquid contact" refers to an arrangement, wherein fluid may transfer from the intake zone of the chassis without significant reduction of the fluid transfer properties, when compared to an arrangement of connecting the two elements to each other under laboratory testing conditions combining the two layers by application of gentle pressure (e.g., as described in urine or menses related acquisition and rewet tests for absorbent pads, see herein below).

The user may don the well-fitting chassis, either with an absorbent pad already inserted or without that. Sometimes, applying the absorbent pad after donning of the chassis may be easier and the pad may be properly positioned upon application.

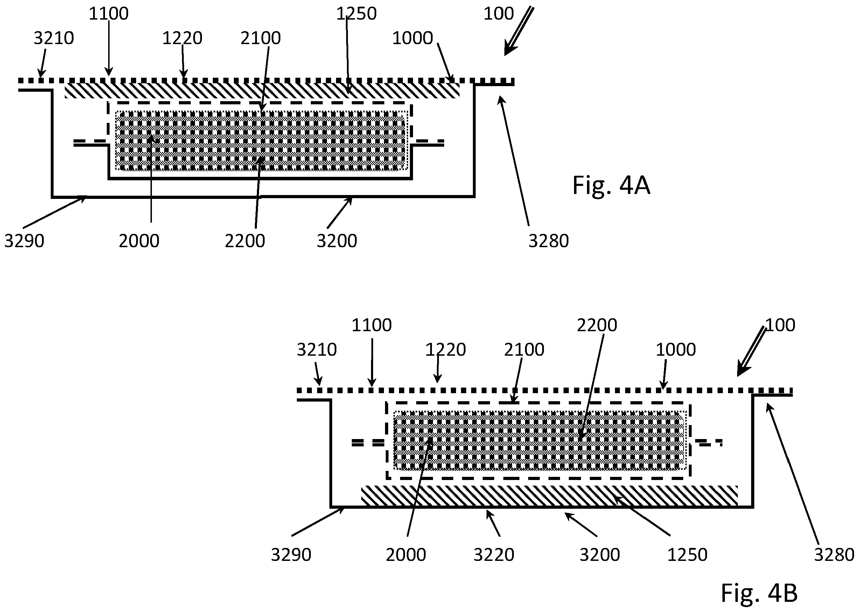

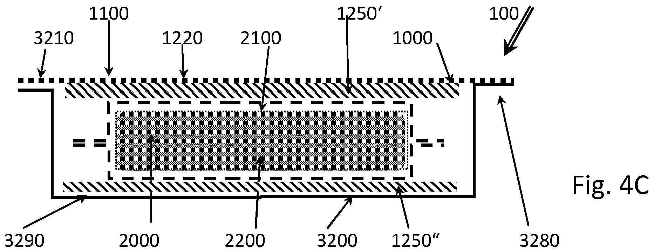

A further particular execution is described by referring to Fig. 4. For this combination 100 of the chassis 1000 and the absorbent pad 2000, the chassis comprises a pocket 3200 adapted to receive the absorbent pad 2000 with a pocket opening 3210, optionally recloseable, and a pocket attachment 3280 to the outer surface of the chassis. Further, the chassis 1000 comprises a chassis fluid handling aid 1250. In a first execution, the chassis fluid handling aid 1250 is connected to or integral with the materials of the intake zone 1220, e.g. positioned opposite of the inner surface layer 1100 of the chassis 1000, see Fig. 4A, allowing fluid to pass through towards the user oriented surface 2100 of the underlying absorbent pad 2000. In a second execution, see Fig. 4B, the chassis fluid handling aid 1250 is connected to or integral with the pocket material 3220, opposite of the liquid impermeable outer surface 3290 of the pocket material, i.e. positioned towards the absorbent pad 2000, once inserted into the pocket and with all of the surface of the absorbent pad being liquid permeable. In a third execution, see Fig. 4C, the chassis comprises two chassis fluid handling aid 1250' and 1250", positioned such that an absorbent pad 2000, with all of its surfaces being liquid permeable, can be sandwiched between these. For any of these executions, the absorbent pad 2000 comprises an absorbent core 2200 that is adapted to absorb liquid from the chassis fluid handling layer, such as by exhibiting a higher capillary suction, preferably further supported by osmotic suction of SAP comprised therein, preferably at a weight ratio of more than about 60% SAP in the absorbent core. A particularly suited execution for the chassis fluid handling aid 1250 may be a high loft acquisition layer, such as available under the designation DRY WEB TDL2, at 60 g/m² from TWE GmbH & Co. KG, Germany. In a preferred execution, the dimensions of the absorbent pad are at least laterally smaller than the ones of the pocket, thereby easing the application and removal of the absorbent pad.

Thus, the system according to the present invention provides particular fluid handling properties, in that it allows a fluid handling aid, as may be positioned in the liquid intake region of the article chassis to receive the liquid rapidly, but also release the liquid to an absorbent pad, even if this exhibits a smaller size than the chassis fluid handling aid.

This can be demonstrated by determining the amount of liquid as being released from a material for a fluid handling aid into a smaller sized absorbent pad, according to the test for "Liquid release of fluid handling aid material to ultimate liquid storage material" as described herein below.

A suitable exemplary material for a fluid handling aid material is commercially available under the trade designation ATB Multifunctional Blue A49B60 from Texsus spa, Italy, at 60 g/m² basis weight. When combined with a 180 g/m² basis weight airlaid absorbent pad material from Glatfelter GmbH, Germany, code MM180 .SMT.CSN00.V03.F.PEFC.S.046w, the fluid handling aid material released 85.1 % of the total fluid pick-up. Similarly, when combined with a 270 g/m² eCore absorbent pad material from Glatfelter Falkenhagen GmbH, Germany, code MM270.PTB.CST00.V00.R.FSCM2x0046w.1050d, the fluid handling aid material released 92.0 % of the total fluid pick up.

This demonstrates that when removing the absorbent pad from the hygiene article chassis, e.g. for replacing it by a fresh absorbent pad, the chassis fluid handling aid is dried out to a significant degree and ready to receive further fluid load for release to the fresh absorbent pad.

### Test methods

All percentages are on a weight basis, unless expressly referred to otherwise.

All testing should be performed in a conditioned room maintained at about 23 +/- 2 °C and about 50 +/- 2 % relative humidity.

"NWSP" refers to the "Nonwovens Standard Procedures", Edition 2015 (suffix R0.15), issued by EDANA, Belgium.
- "Repeated liquid strike through" - NWSP 070.7 & "wet-back after repeated strike through" (rewet) (NWSP 070.8) for nonwoven materials.
- "Water Vapor Transmission Rate" (WVTR) by the principle of measuring the time to increase humidity ("Lyssy") - Part 2, NWSP 070.6.
- "Vertical Wicking of Textiles": using a 0,9% NaCl solution according to AATCC197 of the American Association of Textile Chemists & Colorists (AATCC 197, 2018 Edition, 2018).
- "Air permeability": American Society for Testing and Materials (ASTM) D737-18 at 125 Pa and a surface of 20 cm².
- "pH measurement of cellulose material": Wetting the pulp/core pouring the six fold of its own dry-weight of an aqueous 0.9% NaCl solution on the upper surface of the cellulose and after 5 minutes using a pH indicator paper (Merck 1.09527.0001 or equivalent) to determine the pH by pressing the indicator paper with about 0,02 bar against the wetted surface at the intake point of the cellulose.
- "Absorbent Capacity - Centrifuge Capacity": This test is generally following the EDANA NWSP 241.0.R2 (15) "Polyacrylate Superabsorbent Powders - Determination of the Fluid Retention Capacity in Saline Solution by Gravimetric Measurement Following Centrifugation," but applying it to an absorbent material, as per Test Method PA07/05 of Hygiene-Technologie GmbH (Hy-Tec), Haan, Germany. To this end, a test sample as treated according to the free swell absorption test is positioned within a commercial centrifuge exerting a centrifugal force of about 250 times gravity (e.g. by having an internal diameter of 225 mm at a rotation of 1400 rpm) for 3 mins. The test specimen is removed and weighed and the centrifuge absorbent capacity recorded in grams.
- "Free Swell Absorption Capacity": Following the Test Method PA07/05 of Hygiene-Technologie GmbH (Hy-Tec), Haan, Germany, the test specimen is submersed in sufficient 0.9 w-% saline for 30 mins, after which is removed and placed on a coarse grid or sieve, e.g. a grid with 1 cm wide opening and a ribs of about 2 mm. After a further waiting time of 10 mins, the weight of the test specimen is recorded as free swell absorbency in grams-.
- "Urine related Acquisition and Rewet Determination - absorbent pads"

The Acquisition / Rewet measurement of products designed for use in Adult Incontinence Test Method PA07/03 of Hygiene-Technologie GmbH (Hy-Tec), Haan, Germany.

The test employs an apertured Perspex plate of 70 mm by 220 mm with a central cylinder of an inner diameter of 20 mm and a height of 130 mm at a weight of 255 g, adapted such that weights of 2 kg each can be placed on onto the plate on either side of the cylinder. Upon placing the cylinder and the plate with weights centered on the plate, 50 ml of 0.9 w-% saline as synthetic urine are applied to the cylinder, and the acquisition time until complete disappearance of the liquid is recorded as 1^{st} gush acquisition time. 20 mins after that point in time, the second 50 ml are applied, and the 2^{nd} gush acquisition time until disappearance is recorded. After another 20 mins, the 3^{rd} 50 ml are applied and the 3^{rd} acquisition time is recorded. After 5 mins, the weights, plate and cylinder are removed and accurately (i.e. +/- 0.01 g or better) weighed 50 g of filter paper of 110 mm by 230 mm, are placed on the test specimen, followed directly by applying a plate without cylinder at 145 g and the 2^{∗}2 kg weights. After 15 secs, the weights and plate are removed and the rewetted filter paper is accurately weighed. The three acquisition times are added to the cumulative acquisition time in seconds, and the rewet value is recorded in grams.
- "Menses related Acquisition and Rewet Determination - absorbent pads"

The Acquisition / Rewet measurement for the absorbent pads, which are applied during menstruation is based on the specification of PA 05/02 and PA 05/01 from Hygiene Technologie GmbH (Hy-Tec). The sample is placed horizontal on a plate. A ring out of Teflon (80 ± 1g, inner diameter 30 ± 0.1 mm, height 21.5 mm) is positioned in the middle of the sample. Afterwards, the test fluid artificial menses (solution of demineralized water with 1.4 % Tylose H 20 P2, 0.4 % Sodium Hydrogen Carbonate, 0.5 % Sodium Chloride, 0.025 % Alura Red Dye, 10 % glycerin) is applied from 1 cm above the ring into the ring. The time until the fluid is absorbed into the pad is measured (= acquisition). After 10 minutes, filter paper (type 604, approximately 10 sheets, 140 x 85 mm) is weighed and placed with a weight on top of the wetted sample as described above. After 15 s the weight and the filter paper sheets are removed. The filter paper sheets are weighed again. The increased mass of the filter paper sheets due to fluid uptake is calculated (= rewet).
- "Surface pH measurement of absorbent pad": Gently pressing a pH indicator paper (MERCK 1.09527.0001 or equivalent) at a pressure of about 0,02 bar against the surface of the of the absorbent pad intended to be directed towards the intake zone of the chassis after slowly pouring, avoiding liquid run-off, an amount of half of the free swell absorption capacity (Following the "Free Swell Absorption Capacity Test" Method, as described in the above) of the absorbent pad of an aqueous 0.9% NaCl solution onto the surface of the absorbent pad intended to be directed towards the intake zone of the chassis and waiting for 5 minutes previous to measuring the pH.
- "Topsheet Run-off Test": This test aims at determining the ability of the material to retain liquid applied thereto. This test is based on the EDANA test method Nonwoven Coverstock Run-Off test NWSP 080.9R0 (15), and employs a 10° inclined base plate onto which the test specimen was placed at a length of at least 25 cm extending from the lower end of the plate. At that point 25 ± 0.5 g of 0.9 w% saline was applied through a centered tube with an inner diameter of 5 mm, distanced vertically 25 mm above the web, at a flow rate of 6.3 g/sec. After application of the fluid the weight increase of the web was recorded.
- "Tensile properties": The tensile properties of the materials may be determined according to the NWSP 110.4, for a material width of 25 mm, length 150 mm, a gage length of 75 mm, and an extension speed of 300 mm / min.

- "Basis weight": NWSP 130.1, adapted to respective materials.
- "Single Load Drop Test": At least three drops of de-ionized water / saline, for which contamination by any surface active substance has been avoided, thus exhibiting a surface tension of at least 70 dyn / cm, are placed on a test specimen, such as by a clean pipette. By visual observation it is determined, if these drops are penetrating into the substrate or remain on the surface for at least 60 secs. In the latter case, the test specimen is considered hydrophobic, otherwise hydrophilic.
- "Stiffness of Nonwoven Fabrics Using the Cantilever Test" (INDA) NWSP 090.1.
- "Nonwoven Thickness" The thickness for the samples was determined generally following EDANA Standard Procedure NWSP 120.1, with a circular pressure foot with 3 cm diameter at a pressure of 15g/m².
- "Aperture measurements": The geometric determination of the apertures and their patterns may be determined by any suitable optical method. A particular suitable method may be employed according to the disclosure in EP3215086, to which express reference is made for the aperture related aspects of the "Aperture / Feret Angle Test", wherein aperture dimensions, effective open area and inter-aperture distance measurements are obtained from specimen images acquired using a flatbed scanner using a reflectance mode at a resolution of 6400 dpi and 8 bit grayscale. The scanner is interfaced with a computer running an image analysis program, and calibrated against an acquired image of a ruler certified by NIST. The test specimen may be mounted in a steel frame and be backed with a black glass tile.
- "Combined Chassis / absorbent pad Post Acquisition Rewet Test": This test aims at assessing the acquisition time and rewet in a horizontal position of an absorbent system by combining a Chassis fluid handling aid material of 300 mm x 100 mm with an absorbent pad of 100 mm x 100 mm with the absorbent pad centred under the Chassis fluid handling aid material at the application point of the liquid. The test is performed according to NWSP 070.8.R0 (15) with the modification of replacing the 10 plies of filter paper by the absorbent system and by having a 10 minutes wait period between the gushes. The liquid load was set to 5 ml per gush. The rewet is determined after the 3rd gush and a waiting period of 10 minutes. In case of liquid seeping out at the sides (rather than entering into the material), the sample may be enveloped in a plastic film, e.g. typical backsheet film for absorbent articles, without covering the area over which liquid is applied.
- "Liquid release of fluid handling aid material to ultimate liquid storage material of absorbent pad": A sample of the fluid handling aid is cut into a sheet of a size of 150 mm x 92 mm and fully immersed into a sufficiently sized bath of saline solution (0.9 g/l NaCl) for 10 secs to ensure saturation, whilst placed on a perforated plate with approximately 12 holes per cm² at a hole diameter of 2 mm. Still placed on the perforated plate, the fully soaked sheet is removed from the bath and left horizontally for 5 min to allow excess liquid to drip off. Afterwards, the sheet is transferred on a solid plate out of PMMA or equivalent and weighed to determine the amount of total liquid in the fluid handling aid. A pre-weighed stripe of the absorbent pad material (150 mm x 46 mm) is placed lengthwise in the middle of the soaked fluid handling aid material. A further plate of PMMA or equivalent weighing 140 g is put lengthwise on top of the absorbent pad stripe. After 10 min the absorbent pad is removed and weighed without applying squeezing forces, and the amount of the absorbed liquid in the absorbent pad is calculated. The liquid releasing performance is the amount of the absorbed liquid released from the fluid handling aid and sucked into the absorbent pad material in weight percent, relative to the total amount of fluid picked up by the fluid handling aid material.

## Claims

1. An article chassis for being worn on the lower torso of a wearer exhibiting a user-oriented surface and an opposite outer surface;
said article chassis comprising
- at least one liquid intake zone adapted to cover a region of the exudate releasing body openings, comprising an intake zone material exhibiting, when tested on the surface adapted to be in contact with the user
- a repeated liquid strikethrough value according to NWSP 070.7 R0 (15) of less than 50 sec, preferably less than 25 sec, preferably less than 15 sec and most preferred less than 10 sec and
- a wetback value after repeated liquid strikethrough of less than 10g, preferably less than 5 g, preferably less than 1 g, more preferably less than 0.5 g, even more preferably less than 0.3 g, according to NWSP 070.8 R0 (15);
- a main chassis region preferably circumscribing said at least one liquid intake zone;
- optionally a transition region, connecting intake zone and main chassis region,
wherein at least one of said main chassis and said transition region comprise material exhibiting a vertical capillary wicking height of less than 10 mm, preferably less than 5 mm, more preferably being essentially non-wicking, according to AATCC 197, 2018 Edition, 2018 - Test Method for Vertical Wicking of Textiles, optionally being water repellant or being liquid impermeable,
said article chassis further comprising one or more combination element(s) for combination with an absorbent core on the outer surface in contact with the liquid intake zone, selected from the group consisting of
a) said outer surface being at least partially adapted to engage with mechanical fastener and/or connect with adhesives and/or van-der-Waals forces based micro-structured fastener film;
b) pockets positioned on said outer surface covering at least a portion of at least one intake zone, optionally made of an elastic material, optionally being liquid impermeable at the surface opposite to the intake layer;
c) straps positioned on and permanently connected to said outer surface, optionally being
c1) elastic and permanently connected at both ends
or
c2) permanently connected only at one end and releasably connected on the other end, preferably with mechanical fastener and/or adhesives on the other end and/or van-der-Waals forces based micro-structured fastener film.

2. An article chassis according to claim 1, further comprising one or more elements selected from the group consisting of
d) leg cuffs, preferably at an extension from base to end of more than 1 cm, preferably more than 2 cm, more preferably more than 3 cm;
e) openable side closure elements that are in a closed configuration;
f) consisting essentially of materials and construction elements that are washable at between 40°C and 90 °C;
g) comprise materials exhibiting a Moisture Vapor Transmission Rate (MVTR) of at least 5000 g/m²/24h, preferably more than 8000 g/m²/24h when tested according to "Water Vapor Transmission Rate by the Principle of Measuring the Time to Increase Humidity Lyssy/EDANA," Part 2 NWSP 070.6.R0 (15);
h) comprise materials exhibiting an air permeability of at least 0.2 m³/(m^{2∗}s), preferably more than 1 m³/(m^{2∗}s) when tested according to ASTM D 737-18 measured at 125 Pa with a surface area of 20 cm².

3. An article chassis according to claim 1 or 2, further exhibiting one or more elements selected from the group consisting of
i) a circumferential extensibility at least in a waist region of at least 10%, preferably 50% and more preferably 100% of the unstretched circumference;
j) the one or more intake zone(s) are adapted to male user, female user, or unisex;
k) the materials of the main chassis region and an intake region exhibit a difference of at least 10% of
- repeated liquid strikethrough time (NWSP 070.7 R0 (15));
- wetback after repeated strikethrough time (NWSP 070.8 R0 (15));
- Vertical capillary wicking for 0.9% NaCl solution according to AATCC 197, 2018.

4. A hygiene article system, comprising an article chassis according to any of claims 1 to 3 and an absorbent pad,
said absorbent pad comprising
- a topsheet with a wearer oriented surface;
- an opposite surface with a backsheet:
- an absorbent core in between comprising absorbent pad absorbent material;
- optionally comprising a pad fluid handling aid;
said absorbent pad being adapted
- to be positioned in registry with at least a portion of said intake zone of said article chassis
and
- to be combined with its wearer oriented topsheet surface to said article chassis by means of said combination element(s) of said article chassis.

5. A hygiene article system according to claim 4, wherein said absorbent pad further comprises one or more elements selected from the group consisting of
z) a tab or a handle as removal aid
z1) as an extension of a peripheral region of the pad; or
z2) attached to the outer surface of the pad;
y) a finger pouch on its outer surface as application aid;
x) a removable release cover;
w) cellulosic pulp exhibiting a pH of less than 6, preferably less than 5.5 according to the "pH measurement test of cellulose", as described herein;
v) an odor reducing agents;
u) a bacterial growth inhibitor;
t) absorbent material, optionally superabsorbent material such that said absorbent pad exhibits an absorbent capacity of more than 10 ml, preferably more than 50 ml or more preferably of more than 100 ml of an aqueous 0.9% NaCl solution;
s) absorbent material, optionally superabsorbent material and optionally an acquisition aid, said article exhibiting a rewet value of at less than 10g, preferred less than 5g and most preferred less than 1g when submitted to the "Adult Incontinence Article: Rate of acquisition and Re-Wet test NWSP 070.9 R1 (15)".

6. A hygiene article system according to claim 4 or 5, wherein said absorbent pad further exhibits one or more features selected from the group consisting of
r) a urine related Acquisition value of less than 300 sec, when submitted to the Urine related Acquisition and Rewet Determination Test PA07/03;
q) a urine related Rewet value of less than 10 g, preferably less than 5 g, and more preferably of less than 1 g, when submitted to the Urine related Acquisition and Rewet Determination Test PA07/03;
p) a menses related Acquisition value of at least less than 20 sec when submitted to the Menses related Acquisition and Rewet Determination Test PA 05/02;
o) a menses related Rewet value of less than 5 g when submitted to the Menses related Acquisition and Rewet Determination Test PA 05/01;
n) a surface pH value of less than about 7.0, preferably less than about 5.5, more preferably of less than about 5, when tested according to the surface pH measurement test, as described herein.

7. A hygiene article system according to any of claims 4 to 6, wherein said absorbent pad exhibits a size smaller than said fluid intake zone of said hygiene article chassis, preferably covering less than 90 %, preferably less than 70%, more preferably less than 50% of said fluid intake zone, optionally having a liquid permeable backsheet material.

8. A hygiene article system according to any of claims 4 to 7, wherein said hygiene article chassis comprises a chassis fluid handling aid, positioned
- in direct contact with said liquid intake zone, and/or
- on a user oriented surface of a pocket according to claim 1 b).

9. A hygiene article system according to claim 8, wherein said chassis fluid handling aid is adapted to release more than about 50%, preferably more than about 85%, more preferably more than about 90% of its liquid load to the absorbent pad material, when submitted to the "Liquid release of fluid handling aid material to ultimate liquid storage material of absorbent pad" test, as described herein.
